# EUROPEAN PATENT SPECIFICATION

(11) **EP 4 404 916 B1**
(45) Date of publication and mention of the grant of the patent: **06.08.2025**
(21) Application number: 22790469.5
(22) Date of filing: 16.09.2022
(51) Int. Cl.: A61K 9/08, A61K 9/12, A61P 11/02, A61K 47/36, A61K 31/728, A61K 31/4174

(54) **AN IMPROVED PHARMACEUTICAL COMPOSITION FOR NASAL USE, PREPARATION, AND USE THEREOF**
VERBESSERTE PHARMAZEUTISCHE ZUSAMMENSETZUNG ZUR NASALEN ANWENDUNG, HERSTELLUNG UND VERWENDUNG DAVON
COMPOSITION PHARMACEUTIQUE AMÉLIORÉE POUR UTILISATION NASALE, PRÉPARATION ET UTILISATION DE CELLE-CI

(30) Priority: 22.09.2021 HR 20211486
(43) Date of publication of application: 31.07.2024
(73) Proprietor: JADRAN - GALENSKI LABORATORIJ d.d., 51000 Rijeka (HR)
(72) Inventor: KNEZEVIC, Zdravka, 10000 Zagreb (HR); VUKELIC, Karina, 51215 Kastav (HR); SIMIC, Laura, 51000 Rijeka (HR); POPOVIC, Nina, 51215 Kastav (HR); SARSON, Vesna, 51216 Viskovo (HR)
(74) Representative: Bihar, Zeljko
(86) International application number: PCT/EP2022/075786
(87) International publication number: WO 2023/046590

(56) References cited:
- WO-A1-02/051380
- WO-A2-2019/125330
- CN-A- 104 546 716
- DE-U1- 202012 002 792

## Description

### Field of Invention

The present invention relates to the improved pharmaceutical composition for nasal use based on xylometazoline hydrochloride (1) and low-molecular weight hyaluronic acid (2; LMW HA) or its pharmaceutically acceptable salts such as sodium hyaluronate (2a; LMW SH), the process for its preparation or its use.

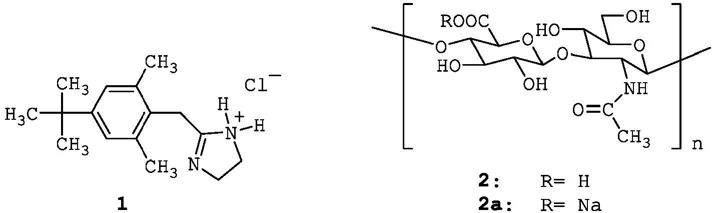

### Technical problem

The technical problem is connected with the disadvantages of liquid pharmaceutical compositions based on xylometazoline hydrochloride (1) which is, as an alpha-adrenergic receptors agonist or as sympathomimetic, commonly used for the topical, intranasal treatment of nasal congestion connected with rhinitis, rhinosinusitis, and sinusitis of various aetiologies, such as:
(a) its toxicity on nasal mucosa cells;
(b) its decreasing effect on mucociliary activity;
(c) inadequate level of mucoadhesive ability; and
(d) limited stability against its hydrolytic degradation to *N*-(2-aminoethyl)-2-[4-(1,1-dimethylethyl)-2,6-dimethylphenyl] acetamide (3):

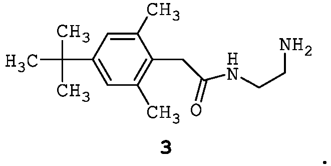

The present invention solves the above-defined technical problem by parallel use of the optimal amount of hyaluronic acid (2) or its pharmaceutically acceptable salt such as sodium hyaluronate (2a) of relative molecular mass (Mᵣ) < 300.000 (or low-molecular-weight hyaluronic acid; LMW HA), which improve liquid pharmaceutical compositions based on xylometazoline hydrochloride (1) in terms of quality, safety and efficacy:
(a) stabilizes xylometazoline against hydrolytic degradation;
(b) provides optimal viscosity of the resulting formulation, which enables sterilization filtration through 0.2 µm filters, which prevents the use of thermal sterilization which would have a negative impact on the hydrolytic stability of xylometazoline;
(c) improves the mucoadhesive property of the resulting formulation; and
(d) stimulates the mucociliary activity of the resulting composition in comparison to the control formulation based on xylometazoline hydrochloride used as a sole API.

### Prior Art

Compound 2-[4-(1,1-dimethylethyl)-2,6-dimethylbenzyl]-4,5-dihydro-1H-imidazole hydrochloride, known under the generic name xylometazoline hydrochloride (1), is a known and widely employed active pharmaceutical ingredient (API) of α-adrenergic activity. It is used as a vasoconstrictor in various indications connected with nasal congestion at rhinitis and sinusitis. In the prior art there is known that the xylometazoline imidazole ring is prone to hydrolysis yielding its degradation product N-(2-aminoethyl)-2-[4-(1,1-dimethylethyl)-2,6-dimethylphenyl]acetamide (3), which is commonly known as xylometazoline "Impurity A"; see, for instance, literature reference 1: 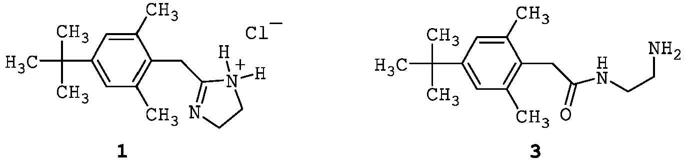
1) European Pharmacopoeia 8.0: Xylometazoline hydrochloride, 01/2008:1162, corrected 7.0, pages 3579-3580.
   Likewise, several different ways of API 1 stabilisation against hydrolysis to impurity A in its liquid formulations is known in the prior art. For instance, stabilisation effects of humectants glycerol (2.0-2.8% w/w), sorbitol (3.5-4.5% w/w), inorganic (phosphate) and organic (tris) buffers, zinc salts (0.1-10% w/w), and panthenol (0.2-10.0% w/w) on chemical stability of xylometazoline in liquid aqueous formulations are known; see literature references 2 -4A:
2) WO 00/78297 A2; U. F. Maerz: Stable xylometazoline and oxymetazoline solution; applicant: Boehringer Ingelheim (DE);
3) US 2006/0222718 A1; F. Boehme, B. Eschenbach, D. Faerber, C. Hey, B. Pfaf, M. Tschaikin: Aqueous pharmaceutical solution containing oxymetazoline and/or xylometazoline; applicant: Merck Patent GmbH (DE);
4) EP 0773022 B3; R. Greve, H. Greve: Pharmaceutical composition for the treatment of rhinitis, containing sympathomimetic and pantothenol and/or pantothenic acid; applicant: Klosterfrau MCM Vertriebs GmbH (DE); 4A) WO 2019/125330 A2; H. Greve: Compositions for nasal application with improved stability.

The solution from literature reference 2 is based on stabilising effect of sorbitol and/or glycerol in combination with inorganic or organic (trometamol) buffer on the hydrolysis of xylometazoline hydrochloride (**1**). The present invention is not based on either glycerol or sorbitol.

The solution from literature reference 3 is based on stabilising the effect of zinc salts such as zinc gluconate and a buffer salt against hydrolytic cleavage of xylometazoline hydrocholoride (**1**) to Impurity A (**3**). The present invention solves this technical problem without any zinc salt.

In the literature reference 4, xylometazoline hydrochloride (**1**; 0.05% w/w) solution was stabilised with panthenol (5% w/w). Herein, the synergic effect of panthenol with API **1** is claimed. The present invention is not based on the use of D-panthenol.

On the other hand, the main disadvantage of xylometazoline in therapy are its negative effects on the ciliary function of the nasal mucosa, and its inhibitory effect on granulocyte chemotaxis. The ciliary function is an important element of mucociliary transport which is responsible for airway clearance and the overall health of the upper respiratory system. In extreme cases, prolonged use of xylometazoline can lead to repeated nasal congestion, rhinitis medicamentosa, and atrophy of the nasal mucosa.

Hyaluronic acid (**2**; HA) is a normal ingredient of the mucus from the nasal mucosa, which by its humectant activity moisturizes and lubricates nasal mucosa and is an important regulator of inflammatory response. Compound **2** in the form of shorter polymeric chains, with low molecular weight <300 kDa (LMW HA), stimulates the proliferation of mucosal cells, and thus stimulates its regeneration and migration of healthy cells which replace those damaged by different reactive oxygen species (ROS), which are generated upon the inflammatory process on the mucosa. Under the influence of the inflammatory process, its fragmentation to very short chains takes place, which stimulates nasal mucosa clearance, or ciliary beat frequency (CBF). Hyaluronic acid (**2**) or its pharmaceutically acceptable salts such as sodium hyaluronate (**2a**) have been used as part of the composition in various topical preparations for intranasal administration. Mostly those products are categorized as Medical Devices used to improve hydration and maintenance of nasal mucosa in a good state, as well as in the treatment of rhinosinusitis; see for instance literature references 5-7:
5) D. Manzanares, M.-E. Monzon, R. C. Savani, M. Salathe: Apical oxidative hyaluronan degradation stimulates airway ciliary beating via RHAMM and RON, Am. J. Respir. Cell Mol. Biol. 37 (2007) 160-168.
6) M. Casale, L. Sabatino, V. Frari, F. Mazzola, R. Dell'Aquilla, P. Baptista, R. Mladina, F. Salvinelli: The potential role of hyaluronan in minimizing symptoms and preventing exacerbations of chronic rhinosinusitis, Am. J. Rhinol. Allergy 28 (2014) 345-348.
7) M. Gelardi, A. V. N. Guglielmi, N. DeCandia, E. Maffezzoni, P. Berardi, N. Quaranta: Effect of sodium hyaluronate on mucociliary clearance after functional endoscopic sinus surgery, Eur. Ann. Allergy Clin. Immunol. 45 (2013) 103-108.

In literature reference 5, the attempt is made to elucidate the mechanism behind HA-stimulated CBF increase by examining the effect of endogenous HA and exogenous high-molecular weight HA (HMWHA). No medicinal product formulation was used during those experiments, nor it was implied that HA might be used as an active pharmaceutical ingredient.

In literature reference 6, patients with chronic rhinosinusitis were treated for 10 days per month (over 3 months) with 9 mg nebulized HA. The product used with HA in its composition was Yabro, IBSA Farmaceutici Italia, a medical device that contains HMWHA, and is used as adjunctive treatment of various respiratory tract diseases in children and adults, with the aim of restoration and maintenance of physiological conditions.

In literature reference 7, patients with grade II nasal polyposis undergoing functional endoscopic sinus surgery were also treated with Yabro^{®}, IBSA (9mg nebulized in 3 mL sodium chloride 0.9%), twice a day, using the Fluirespira nasal douche device. The medical device Yabro has been used in most of the available trials assessing the use of HA in different nasal pathologies, mostly chronic and allergic rhinosinusitis. However, HA present in this product is not an active pharmaceutical ingredient and it also differs in terms of molecular weight, dose, dosage form, and posology from the composition that is disclosed in the present invention.

The compositions are based on a combination of one API xylometazoline hydrochloride (**1**) and hyaluronic acid as an inactive ingredient (excipient) (**2**) or its pharmaceutically acceptable salts such as sodium hyaluronate (**2a**), especially with high-molecular-weight (HMW) HA, have been described in the prior art, e.g., see literature references 8-10:
8) Meralys HA; Summary of Product Characteristics (SmPC); available at HALMED database at the link here: https://www.halmed.hr/en/Lijekovi/Baza-lijekova/Meralys-HA-1-mg-ml-sprej-za-nos-otopina/11419/
9) US 2005/0107330 A1; H. Greve, R. Greve: Pharmaceutical composition for the treatment of rhinitis; applicant: inventors (DE).
10) WO 2019/025040 A1; M. Unkauf, H. Miething: Composition for nasal application; applicant: Maria Clementine Martin Klosterfrau Vertriebs GmbH (DE).

Typical products based on API **1** and HA as an excipient (**2**), like this disclosed in literature reference 8, based on HMW HA. Also, the amount of the excipient used is 0.01 w/w. The composition from invention is not based on HMW HA, but on low-molecular-weight (LMW) **HA (2)** of Mᵣ < 300,000 and with different amounts of LMW HA.

The solution from literature reference 9 is based on the use of a fixed combination of xylometazoline hydrochloride (**1**) and HA or its pharmaceutically acceptable salt, as an excipient. In contrast to the present invention, this document is silent about the stabilising effect of HA (**2**) on the hydrolysis of API **1** to impurity A (**3**).

The solution from literature reference 10 is based on the use of a ternary combination of sympathomimetic such as xylometazoline hydrochloride (**1**), D-panthenol (or pantothenic acid), and hyaluronic acid (**2**) or its salts. This document is considered the closest prior art document to the present invention. It deals with HMW HA of Mᵣ from 600,000-6,000,000. In contrast, the present invention is based on a binary fixed combination of API **1** and LMW HA (**2**) of Mᵣ < 300,000.

The composition based on a combination of two APIs, xylometazoline hydrochloride (**1**) and LMW hyaluronic acid (**2**) or its pharmaceutically acceptable salts, in particularly effective therapeutic concentrations, according to our best knowledge, has not been described for the treatment of rhinitis, sinusitis or rhinosinusitis of various aetiologies, including acute respiratory viral infection (ARVI). Besides this, the composition from the present invention significantly improves liquid pharmaceutical compositions based on xylometazoline hydrochloride as a single API (**1**) in terms of quality, safety, and efficacy:
- increases chemical stability of API **1** against its hydrolytic degradation to impurity A (**3**), which is achieved due to stabilizing effect of LMW HA (**2**)
- improves the mucoadhesive property of the resulting formulation;
- stimulates the mucociliary activity of the resulting composition in comparison to the prior art and control formulations.

In this manner, the above-mentioned complex technical problem is solved by the present invention in a new and inventive way, as is disclosed in section Detailed Description of the Invention.

### Summary of the Disclosure

The present invention discloses an improved pharmaceutical composition for nasal use comprising:
(i) xylometazoline hydrochloride (**1**),
(ii) hyaluronic acid (**2**; HA) or its pharmaceutically acceptable salts such as sodium hyaluronate (**2a**; SH): 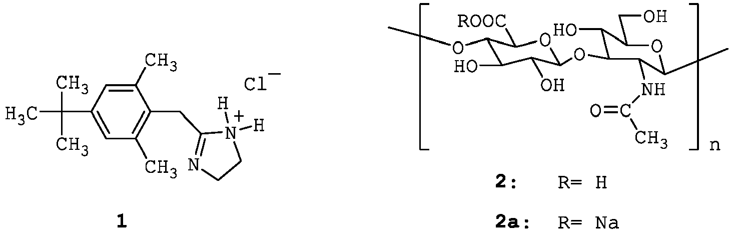 and
(iii) one or more excipients required for the preparation of sterile final dosage forms for nasal use selected from the group consisting of nasal drops, nasal wash, and nasal spray, which are intended for single or multiple uses from the corresponding container;
wherein said formulation exhibits increased stability of xylometazoline and increased mucociliary activity, an adequate level of viscosity not exceeding 10 mPas for efficient sterilisation filtration through 0.2 µm filter and for droplet size formation if used as a nasal spray, osmolality from 0.24-0.50 Osmol/kg and pH value of the said composition selected to be 4.0-7.5, characterised by that the hyaluronic acid (**2**) or its pharmaceutically acceptable salt (**2a**) is used in a low-molecular-weight (LMW HA or SH) form, from 150,000 to 250,000 Daltons (Da).

The pharmaceutical composition according to the present invention contains xylometazoline hydrochloride (**1**) in a mass fraction from 0.05-0.20% w/w, and hyaluronic acid (**2**) or sodium hyaluronate (**2a**) in a mass fraction above 0.01% w/w and not exceeding 1.00% w/w. Preferably, the pharmaceutical composition from the present invention contains two (2) active ingredients, (i) and (ii), and inactive excipients (iii) as given below:
(i) xylometazoline hydrochloride (**1**), 0.05-0.10% w/w,
(ii) sodium hyaluronate (**2a**) equivalent to the content of HA of 0.30-60% w/w, and
(iii) excipients, up to 100% w/w of the formulation.

More preferably, the pharmaceutical composition from the present invention contains two (2) active ingredients, (i) and (ii), and inactive excipients (iii) as given below:
(i) xylometazoline hydrochloride (**1**), 0.10% w/w,
(ii) sodium hyaluronate (**2a**) equivalent to the content of HA of 0.30% w/w, and
(iii) excipients, up to 100% w/w of the formulation.

Pharmaceutical composition from the present invention is used for the production of pharmaceutical products which are employed for the treatment of rhinitis, rhinosinusitis, sinusitis, nasal congestion connected with rhinitis, rhinosinusitis, and sinusitis of different aetiologies including acute respiratory viral infection (ARVI), and for restoring mucociliary activity.

### Description of Figures

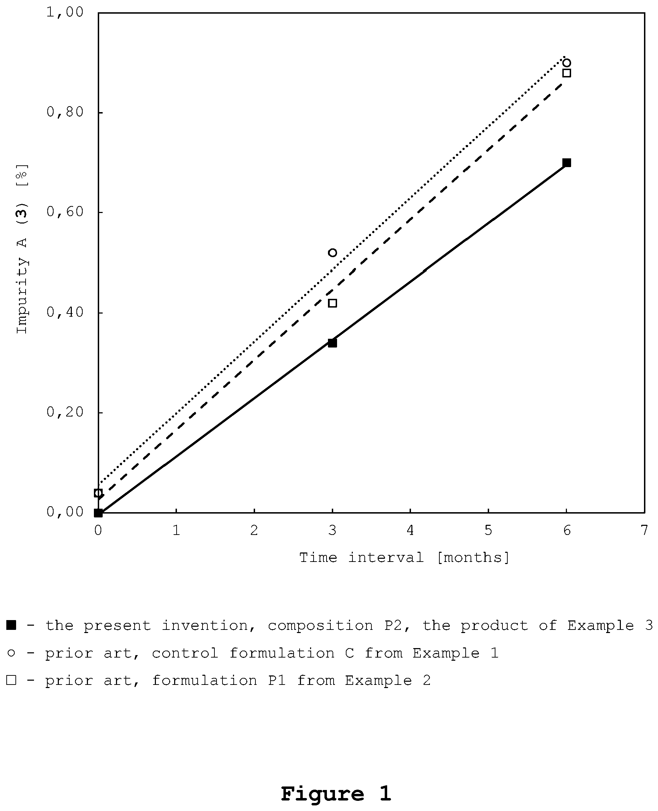

Figure 1 - depicts a graph with key results from the stability study of the composition P2, product of Example 3 (■) from the present invention, in comparison to the control formulation C from Example 1 (∘; prior art) and P1 from Example 2 (□; also prior art) against the kinetic of xylometazoline hydrolytic product N-(2-aminoethyl)-2-[4-(1,1-dimethylethyl)-2,6-dimethylphenyl]acetamide (**3**) formation, which is, according to Ph.Eur., known as impurity A. The limit (1.0%) in the specification for impurity A (**3**) is labelled with a full line.

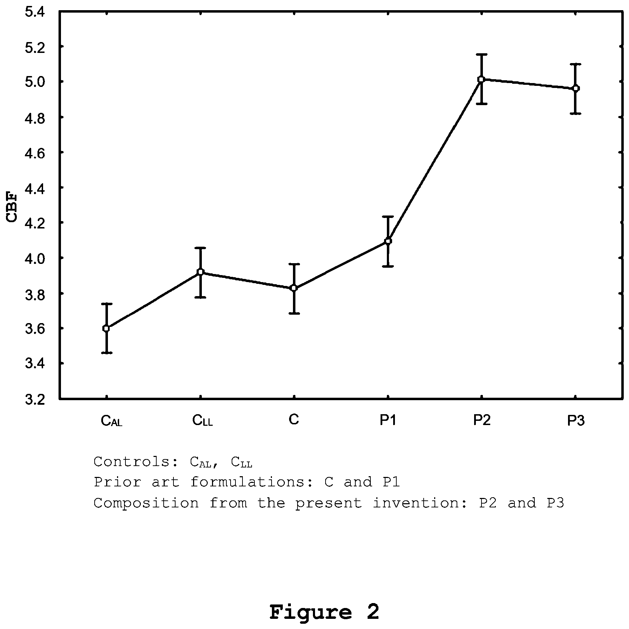

Figure 2 - depicts a graph with a key result of the pre-clinical study of pharmacological effects of the composition from the present invention, products from Examples 3 and 4 (P2 and P3) in comparison to the control formulations C from Example 1 (prior art) and formulation P1 (also prior art), a control on nasal mucosa cells treated with a medium for culture growth of such cells (C_{LL}), and control of nasal mucosa cells without any treatment (CAL), against ciliary beat frequency (CBF). Vertical bars indicate significance intervals of 0.95.

### Detailed Description of the Invention

The present invention discloses an improved pharmaceutical composition for nasal use comprising:
(i) xylometazoline hydrochloride (**1**),
(ii) hyaluronic acid (**2**; HA) or its pharmaceutically acceptable salts such as sodium hyaluronate (**2a**; SH): 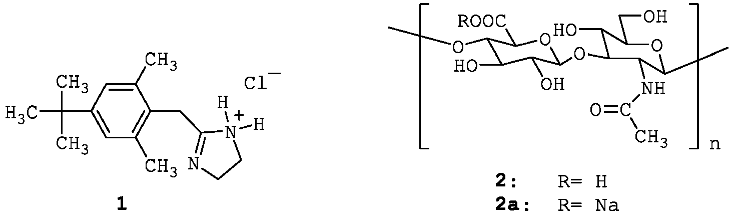 and
(iii) one or more excipients required for the preparation of sterile final dosage forms for nasal use selected from the group consisting of nasal drops, nasal wash, and nasal spray, which are intended for single or multiple uses from corresponding container;
wherein said formulation exhibits increased stability of xylometazoline and increased mucociliary activity, an adequate level of viscosity not exceeding 10 mPas for efficient sterilisation filtration through 0.2 µm filter, and droplet size formation when used as a nasal spray, osmolality from 0.24-0.50 Osmol/kg and pH value of the said composition selected to be 4.0-7.5, characterised by that the hyaluronic acid (**2**) or its pharmaceutically acceptable salt (**2a**) is used in a low-molecular-weight (LMW HA or SH) form, from 150,000 to 250,000 Daltons (Da).

The pharmaceutical composition according to the present invention contains xylometazoline hydrochloride (**1**) in a mass fraction from 0.05-0.20% w/w, and hyaluronic acid (**2**) or sodium hyaluronate (**2a**) in a mass fraction above 0.01% w/w and not exceeding 1.00% w/w.

API **1** as a starting material of pharmaceutical grade quality is used, which conforms to the specification defined by European Pharmacopoeia (Ph. Eur.).

As API **2,** hyaluronic acid (**2**) or its pharmaceutically acceptable salts like sodium hyaluronate (**2a**) of pharmaceutical grade quality are employed, which are commercially available from different manufacturers worldwide.

Preferably, the pharmaceutical composition from the present invention contains two (**2**) active ingredients, (i) and (ii), and inactive excipients (iii) as given below:
(i) xylometazoline hydrochloride (**1**), 0.05-0.10% w/w,
(ii) sodium hyaluronate (**2a**) equivalent to the content of HA of 0.30-60% w/w, and
(iii) excipients, up to 100% w/w of the formulation.

In a more preferred embodiment, the pharmaceutical composition from the present invention contains two (2) active ingredients, (i) and (ii), and inactive excipients (iii) as given below:
(i) xylometazoline hydrochloride (**1**), 0.10% w/w,
(ii) sodium hyaluronate (**2a**) equivalent to the content of HA of 0.30% w/w, and
(iii) excipients, up to 100% w/w of the formulation.

Excipients are selected from the group comprising: purified water, tonicity adjusting agents, humectants, chelating agents, buffer salts, and preservatives.

Preferably, the pharmaceutical composition from the present invention contains excipients selected from the group comprising: purified water, sodium chloride (NaCl), potassium chloride (KCl), calcium chloride (CaCl₂), magnesium chloride (MgCl₂), glycerol, 1,2-propylene glycol, polyethylene glycol 200, polyethylene glycol 300, polyethylene glycol 400, disodium edetate dihydrate (Na₂EDTA•2H₂O), sodium dihydrogenphosphate (NaH₂PO₄) or its monohydrate (NaH₂PO₄•H₂O) or heptahydrate (NaH₂PO₄•7H₂O), potassium dihydrogenphosphate (KH₂PO₄), disodium hydrogenphosphate dihydrate (Na₂HPO₄•2H₂O), dipotassium hydrogenphosphate (K₂HPO₄) or its trihydrate (K₂HPO₄•3H₂O), disodium citrate dihydrate (Na₃C₆H₅O₇•2H₂O), tripotassium citrate monohydrate (K₃C₆H₅O₇•H₂O), citric acid, benzalkonium chloride, chlorhexidine digluconate or dihydrochloride, benzethonium chloride, benzoic acid, sorbic acid, dehydroacetic acid, phenoxyethanol, and benzyl alcohol.

When the formulation is used as a nasal spray, equipped with the dosing device, pharmaceutical composition from the present invention exhibits such characteristics that generate aerosol whose droplet size distribution (DSD) is as given below:

| | |
|---|---|
| - DSD <10>= | >10 µm |
| - DSD <50>= | 20-150 µm |
| - DSD <90>= | <250 µm. |

### Process for the preparation of the composition from the present invention

The process for the preparation of the pharmaceutical composition includes the following manufacturing steps:
(i) dissolution of ingredients (i-iii) in larger part (80-90%) of purified water, by stirring at room temperature during 15-120 minutes;
(ii) addition of the rest of the purified water, up to 100% w/w of the standard manufacturing batch size;
(iii) stirring until the formation of homogeneous solution during 1-30 minutes;
(iv) filtration of thus obtained solution through 1.2 µm filter for elimination of traces of mechanical impurities;
(v) sterilisation of obtained solution via filtration through sterile 0.2 µm filter; and
(vi) filling into sterile containers.

Typical preparations of various versions of the composition are disclosed in Examples 3-6.

### Stability study of the composition from the present invention

For a typical version of the composition from the present invention, P2, whose preparation is disclosed in Example 3, stability testing was performed in comparison to the control formulation C from Example 1 and formulation P1 from the prior art (in line with reference 8) that contains high molecular weight hyaluronic acid (HA; Mᵣ = 1,000,000). Sample were tested under an accelerated stability study according to ICH guidelines at 40 °C and 75% RH during 6 months. The results of this study are presented in Table 1, and Figure 1.

**Table 1. Results of accelerated stability study of formulation P2 from Example 3 according to the present invention against the prior art formulations: control formulation C from Example 1 and formulation P1 from Example 2 at 40 °C/75% RH, see Figure 1.**

| **No.** | **Stability parameter** | **Time interval** | **Product C from Example 1^{a}** | **Product P1 from Example 2^{b}** | **Product P2 from Example 3^{c}** | **Comment** |
|---|---|---|---|---|---|---|
| 1 | pH | T0 | 5.9 | 5.8 | 5.8 | NDT |
| | | T3 | 5.9 | 6.0 | 5.8 | |
| | | T6 | 5.8 | 5.8 | 5.7 | |
| 2 | Osmolality [Osmol/kg] | T0 | 0.300 | 0.295 | 0.280 | NDT |
| | | T3 | 0.302 | 0.295 | 0.281 | |
| | | T6 | 0.302 | 0.298 | 0.282 | |
| 3 | Content of xylometazoline hydrochloride (**1**) | T0 | 99.1 | 101.2 | 99.3 | NDT |
| | | T3 | 98.5 | 101.0 | 98.9 | |
| | | T6 | 99.0 | 100.2 | 99.2 | |
| 4 | Content of sodium hyaluronate (**2a**) | T0 | n.a. | n.a. | 104.1 | NDT |
| | | T3 | n.a. | n.a. | 104.5 | |
| | | T6 | n.a. | n.a. | 104.6 | |
| 5 | Impurity A (**3**) | T0 | <0.04 | <0.04 | ND | IT |
| | | T3 | 0.52 | 0.42 | 0.34 | |
| | | T6 | 0.90 | 0.88 | 0.70 | |
| 6 | Any other impurity | T0 | ND | ND | ND | NDT |
| | | T3 | ND | <0.04 | ND | |
| | | T6 | <0.04 | <0.04 | ND | |

| | | | | | | |
|---|---|---|---|---|---|---|
| T0 = start of the stability study; T3 = after 3 months; T6 = after 6 months; Impurity A = *N*-(2-aminoethyl)-2-[4-(1,1-dimethylethyl)-2,6-dimethylphenyl] acetamide (**3**); ND = not detected; n.a. = not analysed; NDT = statistically significant trend of change of results is not detected; IT = a trend of increasing the content was observed. ^{a} Control formulation C from Example 1 contains xylometazoline hydrochloride (0.1% w/w or 1.0 mg/mL). ^{b} Formulation P1 from Example 2 contains xylometazoline hydrochloride (0.1% w/w or 1.0 mg/mL) and sodium hyaluronate (0.01% w/w or 0.1 mg/mL SH with Mᵣ = 1,000,000) can be considered as a prior art (reference 8). ^{c} Formulation P2 from Example 3 is a typical formulation from the present invention that contains xylometazoline hydrochloride (0.1% w/w or 1.0 mg/mL) and low-molecular weight sodium hyaluronate (0.3% w/w or 3 mg/mL LMW SH). | | | | | | |

All excipients and their concentrations in the control formulation C, formulation P1 from the prior art, and formulation P2 from the present invention are the same.

The results of this study showed significantly increased stability of xylometazoline in the formulation from the present invention due to the presence of 0.3% w/w low-molecular-weight (LMW) hyaluronic acid (**2**). The latter also acts as a stabilising agent which decreases the hydrolysis rate of compound **1** to impurity A (**3**). In this manner, LMW hyaluronic acid (**2**) stabilizes API **1** against its hydrolytic degradation.

The detailed protocol of the stability study of the composition from the present invention is described in Example 7.

### Pre-clinical study of the pharmacological effect of the prior art formulations C and P1, and the compositions P2 and P3 from the present invention on the ciliary beat frequency (CBF)

Pre-clinical study of the composition from the present invention as described in Examples 3 and 4 (formulation P2 and P3) was performed and compared with:
(i) a control formulation C from Example 1 (prior art);
(ii) a control formulation P1 from Example 2 (prior art); in comparison to,
(iii) nasal mucosal cells treated with the medium which is commonly employed for the growth and maintenance of such cell culture (C_{LL}); and
(iv) nasal mucosal cells without any treatment (C_{AL});
against the parameter of ciliary beat frequency (CBF). The reason for the use of additional controls C_{AL} and C_{LL} was due to preliminary testing with MucilAir^{™} cultures and measurement of CBF parameter, where we found that treatment with C_{LL} mildly increases CBF, as well as the basic control with untreated cells (C_{AL}).

The starting hypothesis was that the difference between the CBF parameter of the cultures treated with tested compositions P2 and P3 and untreated (C_{AL}) control will be higher than between tested nasal sprays (P2 and P3) and control treated with a common medium for the maintenance of such cultures (C_{LL}). CBF parameter of the tested compositions P2 and P3 in comparison to P1 was also compared and the hypothesis could not be established based on available prior art. The results of this study are presented in Table 2.

**Table 2. Results of the study of pharmacological effect of the composition from the present invention P2 and P3, from Examples 3 and 4, based on xylometazoline hydrochloride (1) and low-molecular-weight (LMW) hyaluronic acid (2) in comparison to a control formulation C from Example 1 based on 1 without hyaluronic acid (2), control formulation P1 from Example 2 with high-molecular-weight (HMW) as an excipient (0.01% w/w) 2, and additional controls with a medium for the growth and treatment of mucociliary cells (C_{LL}) and control with untreated cells (C_{AL}) in a preclinical model in nasal mucosal cell culture.^{a}**

| **Treatment** | **N** | **CBF M** | **CBF SD** | **CBF SE** | **CBF -95% CI** | **CBF +95% CI** | **CBF %C_{AL}** | **CBF %C**_{**L**L} |
|---|---|---|---|---|---|---|---|---|
| **C_{AL}** | 150 | 3.599 | 0.871 | 0.071 | 3.459 | 3.740 | 100.0 | - |
| **C_{LL}** | 150 | 3.916 | 0.881 | 0.072 | 3.774 | 4.058 | - | 100.0 |
| **C** | 150 | 3.825 | 0.837 | 0.068 | 3.690 | 3.960 | 106.3 | 97.7 |
| **P1** | 150 | 4.093 | 0.939 | 0.077 | 3.942 | 4.245 | 113.7 | 104.5 |
| **P2** | 150 | 5.014 | 0.887 | 0.072 | 4.871 | 5.157 | 139.3 | 128.0 |
| **P3** | 150 | 4.960 | 0.833 | 0.068 | 4.825 | 5.094 | 137.8 | 126.7 |

| | | | | | | | | |
|---|---|---|---|---|---|---|---|---|
| N = number of measurements included in this study; CBF = ciliary beat frequency; M = median value; SD = standard deviation; SE = standard error; CI = confidence interval; C: 1,0 mg/mL **1;** P1: 1,0 mg/mL **1** + 0,1 mg/mL HMW **2** (Mᵣ = 1,000,000); P2: 1,0 mg/mL **1** + 3 mg/mL LMW **2** (Mᵣ < 300,000); P3: 1,0 mg/mL **1** + 6 mg/mL LMW **2** (Mᵣ < 300.000). ^{a} All-tested formulations, C, P1, P2, P3, contained the same concentration of xylometazoline hydrochloride at 0.1% w/w or 1.0 mg/mL. | | | | | | | | |

The results of the study showed that only both tested compositions P2 and P3 from the present invention do provide an increased mucociliary activity which is manifested through significantly increased CBF parameter. The key results of this study are presented in Figure 2, while the details of the study protocol are described in Example 8.

### Clinical study of pharmacological effects of the composition from the present invention P2 from Example 3 in comparison to a prior art formulation C from Example 1 on mucociliary function of nose

In continuation of our research, an open, comparative, randomised, prospective, and multicenter-controlled clinical study on parallel groups was performed with the aim to study the safety and efficacy of:
(i) a tested composition P2 from Example 3 according to the present inventions, based on xylometazoline hydrochloride (**1;** 1 mg/mL) and sodium hyaluronate (**2a;** 3 mg/mL); and
(ii) a control formulation C, based on xylometazoline hydrochloride (**1**; 1 mg/mL; without the addition of hyaluronic acid (**2**); analogous to the product from Example 1;
at patients with acute rhinitis with acute respiratory viral infection (ARVI) in the background.

Targeted population: adults (18-65 years of age).

Group size involved in the study: 69 patients per group; overall 138 patients of both sexes.

Patients with a diagnosis of acute rhinitis connected with acute respiratory viral infection (ARVI) were included in the study in the phase of the disease not older than 36 h. After the examination period, patients were randomised into the ratio 1:1, in the group of treated with the testing composition and in the group with the control formulation C.

Study duration: for each group the treatment was no longer than 7 days, which is in accordance with typical therapeutic treatment for analogous products from the prior art based on xylometazoline hydrochloride (1; 1.0 mg/mL); see for instance literature reference 11:
11) J. Klas, N. Kluz, K. Powowar: Xylometazoline and oxymetazoline - unusual effects of everyday drugs (literature review), J. Educ. Health & Sport 11 (2021) 272-281.

Total study duration in patients was no longer than 11 days.

Intervention into the study: intranasal; 1 application with the nasal spray 3x per day. An interval among repeated applications was at least 6 h. The daily dose was not higher than 3x applications in each nostril.

The targets of the study were:
(i) to evaluate the clinical efficacy of the composition from the present invention, formulation P2;
(ii) to establish safety and tolerability of the formulation P2; and
(iii) to carry out parallel analysis of clinical efficacy, safety and tolerability of the formulation P2 against the control formulation C;
in patients with acute rhinitis connected with ARVI;

### Efficacy evaluation criteria:

The efficacy was confirmed on the basis of the following parameters:

### Primary parameters:

- nasal clearance after 1 h from the first dose (on VAS scale from 0-100 mm);
- dynamics of objections based on normalisation of nasal symptoms evaluation by TNSS scale;
- nose congestion;
- rhinorrhea;
- itching;
- sneezing;
- sleep disorder.

The severity of each symptom was evaluated on 4-point scale as follows:
0 - no symptoms;
1 - mild symptoms;
2 - moderate symptoms;
3 - strong symptoms.

### Secondary (optional) parameters:

- therapy duration;
- dynamics of nasal breathing normalisation with Glyatzel mirror;
- dynamics of mucociliary clearance time determined by saccharine test;
- dynamics of nasal mucosa oedema severity and amount and nature of the secretion by ENT examination and rhinoscopy;
- dynamics of laboratory and instrumental parameters normalisation (rhinoscopy, UAC indicators).

The grading of dynamics of the quality of life was performed by scoring according to special scales and questionnaires.

### The results of primary efficacy measurement:

(1) the composition P2 from the present invention is significantly more effective than the control formulation C according to the dynamics of the nasal symptoms on the TNSS scale. The results showed the following values (p = 0.0066; Mann-Whitney test): P2: -98.08% ± 6.47; C: -94.92% ± 8.82;
(2) the composition P2 from the present invention is not less effective than the control formulation C on the following parameters: analysis of the primary efficacy measurement of "nasal clearance on 1 h after the first dose (on VAS scale from 0-100 mm)" showed that P2 is not less effective than C. The difference between the average values of the tested (P2) and the control (C) groups was 0.68 mm; 95% confidence interval for the indicated mean value difference was [-1.04; 2.41] mm. The upper limit of the specified confidence interval is 2.41 mm; the corresponding value is less than 5 mm.

### Comparison of efficacy by secondary parameters:

(1) a statistically significant difference between the main (P2) and control (C) groups in the mucociliary clearance time dynamics was found, p = 0.0244 (Mann-Whitney test): (average [25 and 75 percentiles] -59.26 [-61.90; -52.38]% and -54.17 [-59.09; - 50.00]% in the main group and the control group, which indicates a significantly enhanced effectiveness of the formulation P2 from the present invention;
(2) a statistically significant difference in swelling of the nasal mucosa at visit (p = 0.0317 and 0.0424; χ2 = Pearson test) and nasal congestion (p = 0.0353 and 0.0452; Pearson χ2 criteria) at visits 4 and 5 was shown, which indicates a significantly enhanced the effectiveness of formulation P2 compared to the control formulation C;
(3) a statistically significant difference in the relative dynamics of "nasal symptoms" was demonstrated: (average ± SD) 96.89 ± 7.26 and -93.85 ± 9.55; p = 0.0457, Mann-Whitney test); which also indicates a significantly increased effectiveness of the formulation P2 in comparison to the control formulation C;
(4) there is no difference between the frequency of early termination of the therapy between groups: p > 0.05, Pearson χ2 test;
(5) dynamics of changes between groups as a function of nasal breathing (condensed spot size, measured with a Glyatzel mirror) were recorded in both tested groups; however, the differences are not statistically significant (p = 0.3499, Mann-Whitney test);
(6) the analysis of the evaluation of the quality of life (QoL) using the MiniRQLQ questionnaire showed a significant improvement in the QoL indicators; in both groups, there are statistically significant differences in the indicators of "activity", "practical problems", and "general symptoms" within the questionnaire, however, these differences are not statistically significant (p > 0.05, Mann-Whitney test);
(7) comparative analysis showed no statistically significant differences between the groups in the CBC parameter "complete blood count" [p > 0.05, Mann-Whitney test (p-level)]

Other details of the clinical study of the composition from the present invention are described in Example 9.

### Use of the composition from the present invention

Pharmaceutical composition from the present invention is used for the production of pharmaceutical products which are employed for the treatment of rhinitis, rhinosinusitis, sinusitis of different aetiologies including acute respiratory viral infection (ARVI), nasal congestion connected with rhinitis, rhinosinusitis, and sinusitis, and for restoring mucociliary activity.

Specifically, the composition from the present invention is for use in the treatment of nasal congestion connected with rhinitis, rhinosinusitis, and sinusitis.

### Examples

### General remarks

All quantitative compositions are expressed as a mass fraction or weight percentages (% w/w) of each particular ingredient within the corresponding composition. The term "room temperature" is related to a temperature interval from 20-25 °C. A stirring rate of a mixing element is expressed in the number of revolutions per minute (r.p.m.). The relative humidity is expressed with an abbreviation RH and expressed as a percentage (%) of relative humidity at a given temperature.

Various grades of sodium hyaluronate (**2a**) were purchased from Contripro a.s., Czech Republic (EU).

Measurement of the droplet size distribution (DSD) of droplets generated during the application of the composition from the present invention in the form of a spray from an adequate dosing device, is conducted with the instrument Malvern Spraytec equipped with a Vereo NSx spray pump for activation under the following conditions:

| | |
|---|---|
| Distance from the laser axis: | 30 mm |
| Focal length of the lens: | 300 mm |
| Laser wavelength: | 632.8 nm |
| Recovery rate: | 50 mm/s |
| Acceleration: | 2.000 mm/s² |
| Time of measurement: | 400 ms |
| Type of activator: | Transmission drop to <80% |
| Time delay of the activator: | 50 ms |

Viscosity is measured on Brookfield LV DV-II+Pro instrument equipped with UL-Y spindle at 160 r.p.m. and measurement during 30 seconds (single point measurement). The temperature of the samples was adjusted to 25.0 ± 0.1 °C. Sample size: 16 ± 0.5 mL.

### Abbreviations:

- ARVI -: active respiratory viral infection;
- CBF -: ciliary beat frequency, a parameter of mucociliary clearance, or the speed of the purification of the nasal mucosa;
- DSD -: droplet size distribution;
- ICH -: International Conference on Harmonisation
- LMW HA -: low-molecular-weight hyaluronic acid (**2**), of Mᵣ < 300.000
- PDA/DAD -: photo-diode array/diode-array detector;
- RH -: relative humidity
- r.p.m. -: revolutions per minute
- SH -: sodium hyaluronate (**2a**)
- TNSS -: Total Nasal Symptom Score; a methodology for quantitative determination or evaluation of the severity of nasal symptoms, e.g., at rhinitis;
- *t*_{R} -: retention factor (in HPLC chromatograms);
- UPLC -: ultra-high-performance liquid chromatography;
- VAS -: Visual Analog Scale; an alternative technique for quantification of the nasal congestion (obstruction); an alternative to rhinomanometry.

### Example 1. Preparation of a control formulation C (prior art) of a nasal spray with 1.0 mg/mL xylometazoline hydrochloride (1) (not according to the claims)

Composition (1000 g solution of a nasal spray):
(1) 0.100% w/w (1.000 g) xylometazoline hydrochloride (**1**)
(2) 0.450% w/w (4.500 g) sodium chloride (NaCl)
(3) 0.825% w/w (8.250 g) potassium dihidrogenphosphate (KH₂PO₄)
(4) 0.119% w/w (1.190 g) disodium hidrogenphosphate dihydrate (Na_{2H}PO₄•2H₂O)
(5) 98.506% w/w (985.060 g) purified water, Ph.Eur.

Preparation: To purified water (900.00 g; 90% w/w), ingredients (1-4) were added and dissolved by stirring at 100-150 r.p.m. at room temperature during 15 minutes. Then, the rest of the purified water (85.06 g; 8.506% w/w) was added, and homogenized by stirring for 5 minutes at room temperature. Thus, obtained solution was filtered through 1.2 µm polypropylene (PP) filter. Then, the solution was sterilized by passing through 0.2 µm polyvinylidene difluoride (PVDF) filter and filled into sterile containers with pumps under aseptic conditions.

The product is a clear, colourless solution, pH = 5.9. Osmolality: 0.300 Osmol/kg. Analysis of droplet size distribution (DSD) at application gave the following result: DSD(10)= 25 µm, DSD(50)= 64 µm, DSD(90)= 133 µm.

### Example 2. Preparation of a nasal spray formulation P1 (prior art) with 1.0 mg/mL xylometazoline hydrochloride (1) and 0.1 mg/mL hyaluronic acid (2) (not according to the claims)

Composition (1000 g solution of a nasal spray):
(1) 0.100% (1.000 g) xylometazoline hydrochloride (**1**)
(2) 0.010% (0.100 g) sodium hyaluronate (**2a**), Mᵣ = 1,000,000
(3) 0.450% (4.500 g) sodium chloride (NaCl)
(4) 0.825% (8.250 g) potassium dihydrogenphosphate (KH₂PO₄)
(5) 0.119% (1.190 g) disodium hidrogenphosphate dihydrate (Na₂HPO₄•2H₂O)
(6) 98.496% (984.960 g) purified water, Ph.Eur.

Preparation: To purified water (900.00 g; 90% w/w), ingredients (1-5) were added and dissolved by stirring at 100-150 r.p.m. at room temperature for 30 minutes. Then, the rest of the purified water (84,96 g; 8,496% m/m) was added, and homogenized by stirring for 10 minutes at room temperature. Thus, the obtained solution was filtered through 1.2 µm polypropylene (PP) filter. Then, the solution was sterilized by passing through 0.2 µm polyvinylidene difluoride (PVDF) filter and filled into sterile containers with pumps under aseptic conditions.

The product is a clear, colourless solution, pH = 5.8. Osmolality: 0.289 Osmol/kg. Analysis of droplet size distribution (DSD) at application gave the following result: DSD(10)= 15 µm, DSD(50)= 34 µm, DSD(90)= 83 µm.

### Example 3. Preparation of a nasal spray formulation P2 with 1.0 mg/mL xylometazoline hydrochloride (1) and 3 mg/mL sodium hyaluronate (2a)

Composition (1000 g solution of a nasal spray):
(1) 0.100% (1.000 g) xylometazoline hidrochloride (**1**)
(2) 0.300% (3.000 g) sodium hyaluronate (**2a**), Mᵣ = 150.000-250.000, Mᵣ (average) = 221.000
(3) 0.450% (4.500 g) sodium chloride (NaCl)
(4) 0.825% (8.250 g) potassium dihydrogenphosphate (KH₂PO₄)
(5) 0.119% (1.190 g) disodium hidrogenphosphate dihydrate (Na₂HPO₄•2H₂O)
(6) 98.206% (982.060 g) purified water, Ph.Eur.

Preparation: To purified water (900.00 g; 90% w/w), ingredients (1-5) were added and dissolved by stirring at 100-150 r.p.m. at room temperature for 30 minutes. Then, the rest of the purified water (82.06 g; 8.206% w/w) was added, and homogenized by stirring for 5 minutes at room temperature. Thus, the obtained solution was filtered through 1.2 µm polypropylene (PP) filter. Then, the solution was sterilized by passing through 0.2 µm polyvinylidene difluoride (PVDF) filter and filled into sterile containers with pumps under aseptic conditions.

The product is a clear, colourless solution, pH = 5.8. Osmolality: 0.280 Osmol/kg. Viscosity: 3.1 mPas. Analysis of droplet size distribution (DSD) at application gave the following result: DSD(10)= 19 µm, DSD(50)= 46 µm, DSD(90)= 115 µm.

### Example 4. Preparation of a nasal spray formulation P3 with 1.0 mg/mL xylometazoline hydrochloride (1) and 6 mg/mL sodium hyaluronate (2a)

Composition (1000 g solution of a nasal spray):
(1) 0.100% (1.000 g) xylometazoline hidrochloride (**1**)
(2) 0.600% (6.000 g) sodium hyaluronate (**2a**), Mᵣ = 150.000-250.000, Mᵣ (average) = 221.000
(3) 0.450% (4.500 g) sodium chloride (NaCl)
(4) 0.825% (8.250 g) potassium dihydrogenphosphate (KH₂PO₄)
(5) 0.119% (1.190 g) disodium hidrogenphosphate dihydrate (Na_{2H}PO₄•2H₂O)
(6) 97.906% (979.060 g) purified water, Ph.Eur.

Preparation: To purified water (900.00 g; 90% w/w), ingredients (1-5) were added and dissolved by stirring at 100-150 r.p.m. at room temperature for 30 minutes. Then, the rest of purified water (79.06 g; 7.906% w/w) was added, and homogenized by stirring for 5 minutes at room temperature. Thus, the obtained solution was filtered through 1.2 µm polypropylene (PP) filter. Then, the solution was sterilized by passing through 0.2 µm polyvinylidene difluoride (PVDF) filter and filled into sterile containers with pumps under aseptic conditions. The product is a clear, colourless solution, pH = 5.7. Osmolality: 0.285 Osmol/kg. Viscosity: 7.4 mPas. Analysis of droplet size distribution (DSD) at application gave the following result: DSD(10)= 25 µm, DSD(50)= 67 µm, DSD(90)= 150 µm.

### Example 5. Preparation of nasal drops formulation with 1.0 mg/mL xylometazoline hydrochloride (1) and 3 mg/mL sodium hyaluronate (2a)

Composition (1000 g solution of a nasal drops):
(1) 0.100% (1.000 g) xylometazoline hidrochloride (1)
(2) 0.300% (3.000 g) sodium hyaluronate (**2a**), Mᵣ = 150.000-250.000, Mᵣ (average) = 221.000
(3) 0.450% (4.500 g) sodium chloride (NaCl)
(4) 0.825% (8.250 g) potassium dihidrogenphosphate (KH₂PO₄)
(5) 0.119% (1.190 g) disodium hidrogenphosphate dihydrate (Na₂HPO₄•2H₂O)
(6) 98.206% (982.060 g) purified water, Ph.Eur.

Preparation: To purified water (900.00 g; 90% w/w), ingredients (1-5) were added and dissolved by stirring at 100-150 r.p.m. at room temperature for 30 minutes. Then, the rest of purified water (82.06 g; 8.206% w/w) was added, and homogenized by stirring for 10 minutes at room temperature. Thus, the obtained solution was filtered through 1.2 µm polypropylene (PP) filter. Then, the solution was sterilized by passing through 0.2 µm polyvinylidene difluoride (PVDF) filter and filled into sterile containers with pumps under aseptic conditions. The product is a clear, colourless solution, pH = 5.8. Osmolality: 0.280 Osmol/kg. Viscosity: 7.4 mPas. Analysis of droplet size distribution (DSD) at application gave the following result: DSD(10)= 19 µm, DSD(50)= 46 µm, DSD(90)= 115 µm.

### Example 6. Preparation of nasal wash formulation with 0.5 mg/mL xylometazoline hydrochloride (1) and 3 mg/mL sodium hyaluronate (2a) (not according to the claims)

Composition (1000 g solution of nasal wash):
(1) 0.050% (0.500 g) xylometazoline hidrochloride (**1**)
(2) 0.300% (3.000 g) sodium hyaluronate (**2a**), Mᵣ = 80.000-100.000, Mᵣ (average) = 91.000
(3) 0.450% (4.500 g) sodium chloride (NaCl)
(4) 0.194% (1.940 g) citric acid monohydrate (C₆H₈O₇•H₂O)
(5) 0.563% (5.630 g) disodium hidrogenphosphate dihydrate (Na₂HPO₄•2H₂O)
(6) 0.100% (1.000 g) disodium edetate dihydrate (Na₂EDTA•2H₂O)
(7) 98.343% (983.430 g) purified water, Ph.Eur.

Preparation: To purified water (900.00 g; 90% w/w), ingredients (1-5) were added and dissolved by stirring at 100-150 r.p.m. at room temperature for 30 minutes. Then, the rest of purified water (83.43 g; 8.343% w/w) was added, and homogenized by stirring for 10 minutes at room temperature. Thus, the obtained solution was filtered through 1.2 µm polypropylene (PP) filter. Then, the solution was sterilized by passing through 0.2 µm polyvinylidene difluoride (PVDF) filter and filled into sterile containers with pumps under aseptic conditions. The product is a clear, colourless solution, pH = 5.8. Analysis of droplet size distribution (DSD) at application gave the following result: DSD(10)= 19 µm, DSD(50)= 46 µm, DSD(90)= 115 µm.

### Example 7. Stability study of the composition from the present invention P2 from Example 3 against a control formulation from Example 1 with focus on the formation of hydrolytic product, impurity A (3)

A typical variant of the composition from the present invention P2, whose preparation is disclosed in Example 3, was tested for stability against prior art formulations: an analogous control formulation C from Example 1 and formulation P1 as disclosed in Example 2. The samples were subjected to an accelerated stability study according to ICH guidelines at 40 °C and 75% RH for 6 months.

### Analytical method for determination of content of xylometazoline and impurity A (3)

The content of xylometazoline hydrochloride (1) was determined by the use of ultra-high-performance liquid chromatography (UPLC) with photo-diode array/diode-array detector (PDA/DAD) at 220 nm with a detection option between 200-400 nm. The column was fluorophenyl derived stationary phase with reverse-phase mode, at 30 °C, with a flow of 0.5 mL/min, and a run time 9 min. The mobile phase consisted of phase A and phase B of the following composition:
(i) mobile phase A: phosphate buffer, pH = 2.5 : acetonitrile = 90 : 10, V/V;
(ii) mobile phase B: phosphate buffer, pH = 2.5 : acetonitrile = 30 : 70, V/V;
where the phosphate buffer was 10 mM potassium dihydrogenphosphate (KH₂PO₄) solution, pH = 2.5, and the composition of mobile phase was gradually modified as follows:

| **Time [min]** | **0** | **0.1** | **5.1** | **5.2** | **7.1** | **7.2** | **9** |
|---|---|---|---|---|---|---|---|
| A [% V/V] | 100 | 100 | 66,7 | 0 | 0 | 100 | 100 |
| B [% V/V] | 0 | 0 | 33,3 | 100 | 100 | 0 | 0 |

A commercially available analytical standard of xylometazoline hydrochloride (**1**) was employed as a standard for the preparation of the reference solution.

Samples and reference solution were dissolved in solvent A up to a theoretical xylometazoline concentration of 0.04 mg/mL. For the calculation of xylometazoline content, areas of the reference solution and test samples were compared.

For analysis of impurity A (**3**) the same method was employed as for the determination of xylometazoline content, but the run time was extended from 9 to 11 minutes. Within this extended run time, the composition of the mobile phase was the same as for xylometazoline determination at 9 minutes. As a standard, the commercially available analytical standard of compound **3** was used as a primary standard. Sample solutions and reference substances solutions were prepared in solvent A as follows:
(1) reference solution: xylometazoline hydrochloride (**1**) solution of 0.005 mg/mL concentration; for determination of quantitation limit (LOQ), a solution of xylometazoline hydrochloride (**1**) from the same sample of reference standard batch was prepared at a concentration of 0.00025 mg/mL;
(2) solution of impurity A (**3**) was prepared from a sample of the reference standard of the same compound at concentration of 0.025 mg/mL;
(3) solution for system suitability determination was prepared from the standard of compound **1** in concentration 0.0008 mg/mL and standard of impurity A (**3**) in concentration 0.0005 mg/mL;
(4) placebo solution was of the composition: 3.00 or 6.00 mg/mL sodium hyaluronate, 4.50 mg/mL sodium chloride (NaCl), 8.25 mg/mL potassium dihydrogenphosphate (KH₂PO₄), 1.19 mg/mL disodium hydrogenphosphate (Na₂HPO₄•2H₂O) in purified water (up to 100%); a solution of 0.5 mg/mL concentration was prepared by dilution of placebo solution, 5.0 mL in 50 mL purified water;
(5) a sample solution of 0.1 mg/mL concentration was prepared by dilution of the test solution, 5.0 mL in 50 mL purified water;
(6) LOQ solution was prepared by dilution of 1.0 mL reference solution up to 20.0 mL with a solvent and homogenised by shaking.

The content of impurity A (**3**) was determined by discarding all the peaks that appear in the chromatogram of the placebo solution, and the areas under the peaks in the reference solution and the test sample were compared and multiplied by the correction factor as stated below:

| **Peak** | ***t*_{R} [min] ^{a}** | **RRT^{b}** | **CF** |
|---|---|---|---|
| Xylometazoline (**1**) | ~2.84 | - | - |
| Impurity A (**3**) | ~3.14 | ~1.11 | ~1.52 |
| Other impurities | - | - | 1.00 |

| | | | |
|---|---|---|---|
| ^{a} Retention time (*t*_{R}) expressed in minutes; may vary depending on column batch; ^{b} Relative retention time (RRT) which is calculated against xylometazoline retention time; CF = correction factor; Impurity A (**3**) = *N*-(2-aminoethyl)-2-[4-(1,1-dimethylethyl)-2,6-dimethylphenyl] acetamide. | | | |

### Analytical method for determination of hyaluronic acid (2) assay

The same UPLC device with said DAD detector was employed for this analysis, at a wavelength of 205 nm, with C18 reverse-phase column, at a temperature of 25 °C, with a run time of 27 minutes. The mobile phase consisted of two solvents, A and B, of the following composition:
(i) solvent A: methanol : phosphate buffer, pH = 6.2 = 40 : 60 % V/V; and
(ii) solvent B: acetonitrile : phosphate buffer, pH = 6.2 = 50 : 50 % V/V;
with the following gradient composition of the resulting mobile phase and employed flows:

| **Time [min]** | **0** | **3** | **3.5** | **12** | **13** | **27** |
|---|---|---|---|---|---|---|
| A [% V/V] | 100 | 100 | 0 | 0 | 100 | 100 |
| B [% V/V] | 0 | 0 | 100 | 100 | 0 | 0 |

| **Time [min]** | **0** | **3** | **3.5** | **16** | **16.5** | **27** |
|---|---|---|---|---|---|---|
| **Flow [mL/min]** | 0.5 | 0.5 | 0.75 | 0.75 | 0.5 | 0.5 |

The phosphate buffer was 0.05 mM potassium dihydrogenphosphate (KH₂PO₄) solution of pH = 6.2. A solution of potassium hydroxide R of 450 g/L concentration in water for chromatography was prepared. As a standard for the preparation of reference solution, sodium hyaluronate (**2a**) with molecular weight (MW) of 150.000-250.000 Da (150-250 kDa), with declared **2a** content.

The sample solution and reference solution were prepared in R water for chromatography so that the theoretical sodium hyaluronate (**2a**) concentration was 0.045 mg/mL. To calculate the content of sodium hyaluronate (**2a**), the areas under the corresponding peaks of reference and test samples solutions were compared.

Key results of the stability study of the composition from the present invention are presented in Table 1.

### Example 8. Pre-clinical study of the pharmacological effect of the prior art formulations C and P1, and the compositions P2 and P3 from the present invention on the ciliary beat frequency (CBF)

Pre-clinical study of the composition from the present invention analogous to the products from Examples 3 and 4 (P2 and P3) was performed in comparison to prior art compositions:
(i) a control formulation C from Example 1 (prior art);
(ii) a control formulation P1 from Example 2 (prior art); and, in comparison to,
(iii) nasal mucosal cells treated with a medium that is commonly employed for the growth and maintenance of such cell culture (C_{LL}); and
(iv) nasal mucosal cells without any treatment (C_{AL});
where the parameter ciliary beat frequency (CBF) was determined.

The aim of this study was to confirm the assumed positive influence of hyaluronic acid (**2**) on mucociliary activity compared to xylometazoline (**1**) itself and to find the appropriate dose (concentration) of hyaluronic acid in the formulation. For this purpose, the MucilAir^{™} *in vitro* model was used. It represents a 3D model of human epithelium that is prepared from cells originating from different donors and was developed for respiratory pharmacology research.

The results of this study are presented in Table 2.

### Example 9. Clinical study of pharmacological effects of the composition from the present invention P2 from Example 3 in comparison to a control formulation C from Example 1 on mucociliary function of nose

An open, comparative, randomised, prospective, and multicenter-controlled clinical study on parallel groups was performed to study the safety and efficacy of:
(i) a tested composition P2 from Example 3 according to the present inventions, based on xylometazoline hydrochloride (**1**; 1 mg/mL) and sodium hyaluronate (**2a**; 3 mg/mL); and
(ii) a control formulation C, based on xylometazoline hydrochloride (**1**; 1 mg/mL; without the addition of hyaluronic acid (**2**); analogous to the product from Example 1;
at patients with acute rhinitis with acute respiratory viral infection (ARVI) in the background.

Targeted population: adults (18-65 years of age).

Group size involved in the study: 69 patients per group; overall 138 patients of both sexes.

Patients with a diagnosis of acute rhinitis connected with acute respiratory viral infection (ARVI) were included in the study in the phase of the disease not older than 36 h. After the examination period, patients were randomised into the ratio of 1:1 in the group treated with the testing composition and in the group with the control formulation C.

Study duration: for each group the treatment was no longer than 7 days, what is in accordance with typical therapeutic treatment for analogous products from the prior art based on xylometazoline hydrochloride (**1;** 1.0 mg/mL); see for instance literature reference 11. The total study duration in patients was no longer than 11 days.

Intervention into the study: intranasal; 1 application with the nasal spray 3x per day. An interval among repeated applications was at least 6 h. The daily dose was not higher than 3x applications in each nostril.

Inclusion criteria: written consent of the patient included in the study; patients aged 18-65 years, of both sexes; diagnosis of "acute rhinitis" according to clinical indications that the underlying cause is ARVI; nasal clearance not less than 80 mm according to VAS 0-100 mm; presence of at least two (2) ARVI symptoms: cough, runny nose, headache, pain in muscles or eyeballs, weakness, fever or chills; duration of illness not longer than 36 h; the sum of points on the TNSS scale 4 or less; patients who did not receive therapy with antiallergic or antibacterial drugs within previous 24 h; consent to the use of suitable contraceptive methods from the moment of examination during the study, until the moment of its completion; the patient understands the requirements of the study and agrees to all the restrictions it requires; and a negative pregnancy test.

Exclusion criteria: allergy and suspicion of allergy to any of the ingredients of the composition or similar products; allergic and/or chronic rhinitis; ophthalmic symptoms (itching, pain or watery eyes); confirmed bacterial aetiology of rhinitis; chronic, vasomotor and atrophic rhinitis; suspected meningococcal infection; temperature above 39 °C; injuries of the nose, polyposis, curvature of the nasal septum or other anomalies of the nasal cavity or nasopharynx; asthma; condition after meningeal surgery; hypophysectomy or other trans-nasal operations; participation in studies with other drugs at the same time or within 90 days before the start of the present study; pregnancy or breastfeeding; predisposition to nosebleeds, cystic fibrosis, paralytic intestinal obstruction, sensitivity to adrenergic drugs; the necessity of taking drugs that are not approved by the protocol of this study; acute productive mental symptoms: psychosis, delirium, hallucinations; hyperthyroidism; arterial hypertension, tachycardia, history of ischemic heart disease, severe atherosclerosis; increased intraocular pressure, glaucoma; hyperplasia of the prostate; porphyria; history of chronic alcoholism, addiction to drugs or other chemical agents; hesitancy to enter the clinical trial; inability or unwillingness to understand the rules for participating in the study; serious, decompensated or unstable somatic disease: cirrhosis of the liver, HIV infection, syphilis, hepatitis B and C, diabetes, urethral stenosis, prostatic hyperplasia, or any other disease or condition, which may cause, in the opinion of the researcher, a difficult interpretation of treatment results during the study.

### Industrial Applicability

The present invention is employed for the production of the pharmaceutical composition for nasal use with several very important indications for use. Therefore, the industrial applicability of the present invention is obvious.

## Claims

1. An improved pharmaceutical composition for nasal use comprising two active ingredients (i) and (ii):
(i) xylometazoline hydrochloride <**1**>,
(ii) hyaluronic acid <2; HA> or its pharmaceutically acceptable salts such as sodium hyaluronate <**2a**; SH>: 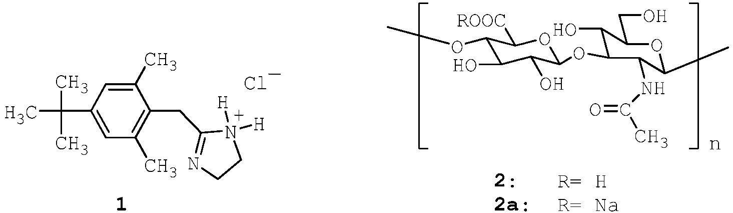 and,
(iii) one or more excipients required for the preparation of sterile final dosage forms for nasal use selected from the group consisting of: nasal drops, nasal wash, and nasal spray intended for single or multiple use from the corresponding container;
wherein said formulation exhibits increased stability of xylometazoline and increased mucociliary activity, osmolality from 0.24-0.50 Osmol/kg and pH value of the said composition selected to be 4.0-7.5, **characterised by that** the hyaluronic acid <**2**> or its pharmaceutically acceptable salt 2a is used in a low-molecular-weight <LMW HA or SH> form, from 150,000-250,000 Daltons.

2. The pharmaceutical composition according to claim 1, wherein it is **characterised by** viscosity not exceeding 10 mPas, measured by rotary viscosimeter at 160 r.p.m. using Y spindle in the sample volume of 16 mL at 25°C, for efficient sterilisation filtration through 0.2 µm filter and for droplet size formation if used as a nasal spray.

3. The pharmaceutical composition according to any of the previous claims, wherein the mass fraction of xylometazoline hydrochloride <**1**> is selected to be 0.05-0.20% w/w, preferably from 0.05-0.10% w/w.

4. The pharmaceutical composition according to any of the previous claims, wherein the mass fraction of hyaluronic acid <**2**> or sodium hyaluronate <**2a**> is selected to be above 0.01% and not exceeding 1.00 % w/w, preferably from 0.30-0.60% w/w.

5. The pharmaceutical composition according to any of the previous claims, wherein the mass fractions of said ingredients (i-iii) are selected to be:
(i) xylometazoline hydrochloride <**1**>, 0.10% w/w,
(ii) sodium hyaluronate <**2a**>, equivalent to the content of HA of 0.30-0.60% w/w, and,
(iii) excipients, up to 100% of the composition.

6. The pharmaceutical composition according to any of the previous claims, wherein excipients are selected from the group comprising purified water, tonicity adjusting agents, humectants, chelating agents, buffer salts, and preservatives.

7. The pharmaceutical composition according to claim 5, wherein excipients are selected from purified water, sodium chloride <NaCl>, potassium chloride <KCl>, calcium chloride <CaCl₂>, magnesium chloride <MgCl₂>, glycerol, 1,2-propylene glycol, polyethylene glycol 200, polyethylene glycol 300, polyethylene glycol 400, disodium edetate dihydrate <Na₂EDTA•2H₂O>, sodium dihydrogenphosphate <NaH₂PO₄> or its monohydrate <NaH₂PO₄ •H₂O> or heptahydrate <NaH₂PO₄•7H₂O>, potassium dihydrogenphosphate <KH₂PO₄>, disodium hydrogenphosphate dihydrate <Na₂HPO₄•2H₂O>, dipotassium hydrogenphosphate <K₂HPO₄> or its trihydrate <K₂HPO₄•3H₂O>, disodium citrate dihydrate <Na₃C₆H₅O₇•2H₂O>, tripotassium citrate monohydrate <K₃C₆H₅O₇•H₂O>, citric acid, benzalkonium chloride, chlorhexidine digluconate or dihydrochloride, benzethonium chloride, benzoic acid, sorbic acid, dehydroacetic acid, phenoxyethanol, and benzyl alcohol.

8. The pharmaceutical composition according to any of the previous claims, wherein, the composition is:
(i) xylometazoline hydrochloride <**1**>, 0.10% w/w,
(ii) sodium hyaluronate <**2a**>, equivalent to the content of HA of 0.30% w/w, and
(iii) excipients are selected to be: sodium chloride <NaCl> 0.45% w/w, potassium dihydrogenphosphate <KH₂PO₄> 0.825% w/w, disodium hydrogenphosphate dihydrate <Na₂HPO₄•2H₂O> 0.119% w/w, and purified water, up to 100% of the composition.

9. The pharmaceutical composition according to any of the previous claims in the final dosage form of a nasal spray for producing an aerosol with, wherein the droplet size distribution <DSD> are:
| | | |
|---|---|---|
| A. | DSD <10> = | >10 µm |
| B. | DSD <50> = | 20-150 µm, and |
| C. | DSD <90> = | <250 µm. |

10. A process for the preparation of a pharmaceutical composition according to any of claims 1-9, wherein the said process includes the following manufacturing steps:
(i) dissolution of ingredients (i-iii) in larger part (80-90%) of purified water, by stirring at room temperature during 15-120 minutes;
(ii) addition of the rest of the purified water, up to 100% w/w of the standard manufacturing batch size;
(iii) stirring until the formation of homogeneous solution during 1-30 minutes;
(iv) filtration of thus obtained solution through 1.2 µm filter for elimination of traces of mechanical impurities;
(v) sterilisation of obtained solution via filtration through sterile 0.2 µm filter; and
(vi) filling into sterile containers.

11. The pharmaceutical composition according to any of claims 1-9, for use in the treatment of rhinitis, rhinosinusitis, sinusitis of different aetiologies including acute respiratory viral infection <ARVI>, nasal congestion connected with rhinitis, rhinosinusitis and sinusitis, and for restoring mucociliary activity.

12. The pharmaceutical composition according to claim 11, for use in particular for treatment of nasal congestion connected with rhinitis, rhinosinusitis and sinusitis.

## Patentansprüche

1. Verbesserte pharmazeutische Zusammensetzung zur nasalen Verwendung, die zwei Wirkstoffe (i) und (ii) umfasst:
(i) Xylometazolinhydrochlorid <1>,
(ii) Hyaluronsäure <2; HA> oder ihre pharmazeutisch verträglichen Salze, wie Natriumhyaluronat <2a; SH>: 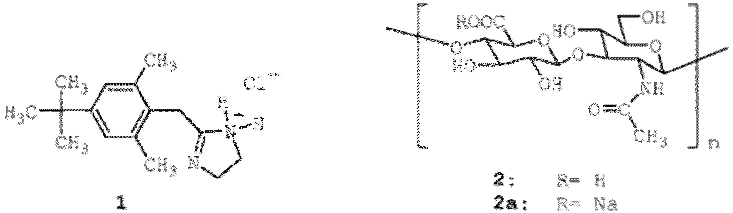 und,
(iii) einen oder mehrere Hilfsstoffe, die für die Herstellung steriler abschließender Dosierungsformen zur nasalen Verwendung erforderlich sind, ausgewählt aus der Gruppe bestehend aus: Nasentropfen, Nasenspülungen und Nasensprays, die zur einmaligen oder mehrmaligen Verwendung aus dem entsprechenden Behälter bestimmt sind;
wobei die Formulierung eine erhöhte Stabilität von Xylometazolin und eine erhöhte mukoziliäre Aktivität, Osmolalität von 0,24-0,50 Osmol/kg darlegt, und der pH-Wert der Zusammensetzung ausgewählt ist, um 4,0-7,5 zu sein, **dadurch gekennzeichnet, dass** die Hyaluronsäure <2> oder ihr pharmazeutisch verträgliches Salz 2a in einer niedermolekulargewichtigen <LMW HA- oder SH>-Form, von 150.000-250.000 Dalton verwendet wird.

2. Pharmazeutische Zusammensetzung nach Anspruch 1, wobei sie durch eine Viskosität gekennzeichnet ist, die 10 mPas, mit einem Rotationsviskosimeter bei 160 U/min unter Verwendung einer Y-Spindel in einem Probenvolumen von 16 ml bei 25 °C gemessen, nicht überschreitet, zur effizienten Sterilisationsfiltration durch einen 0,2-µm-Filter und für die Tröpfchengrößenbildung bei Verwendung als Nasenspray.

3. Pharmazeutische Zusammensetzung nach einem der vorstehenden Ansprüche, wobei der Massenanteil an Xylometazolinhydrochlorid <1> ausgewählt ist, um 0,05-0,20 Gew.-%, bevorzugt 0,05-0,10 Gew.-%, zu sein.

4. Pharmazeutische Zusammensetzung nach einem der vorstehenden Ansprüche, wobei der Massenanteil an Hyaluronsäure <2> oder Natriumhyaluronat <2a> ausgewählt ist, um über 0,01 % zu liegen und 1,00 Gew.-%, bevorzugt 0,30-0,60 Gew.-% nicht zu überschreiten.

5. Pharmazeutische Zusammensetzung nach einem der vorstehenden Ansprüche, wobei die Massenanteile der Inhaltsstoffe (i-iii) dazu ausgewählt sind, wie folgt zu sein:
(i) Xylometazolinhydrochlorid <1>, 0,10 Gew.-%,
(ii) Natriumhyaluronat <2a>, äquivalent mit einem HA-Gehalt von 0,30-0,60 Gew.-%, und
(iii) Hilfsstoffe, bis zu 100 % der Zusammensetzung.

6. Pharmazeutische Zusammensetzung nach einem der vorstehenden Ansprüche, wobei die Hilfsstoffe aus der Gruppe ausgewählt sind, die gereinigtes Wasser, Tonizitätsanpassungsmittel, Feuchthaltemittel, Chelatbildner, Puffersalze und Konservierungsmittel umfasst.

7. Pharmazeutische Zusammensetzung nach Anspruch 5, wobei die Hilfsstoffe aus gereinigtem Wasser, Natriumchlorid <NaCl>, Kaliumchlorid <KCl>, Calciumchlorid <CaCl₂>, Magnesiumchlorid <MgCl₂>, Glycerin, 1,2-Propylenglykol, Polyethylenglykol 200, Polyethylenglykol 300, Polyethylenglykol 400, Dinatriumedetat-Dihydrat <Na₂EDTA•2H₂O>, Natriumdihydrogenphosphat <NaH₂PO₄> oder seinem Monohydrat <NaH₂PO₄•H₂O> oder Heptahydrat <NaH₂PO₄•7H₂O>, Kaliumdihydrogenphosphat <KH₂PO₄>, Dinatriumhydrogenphosphat-Dihydrat <Na₂HPO₄•2H₂O>, Dikaliumhydrogenphosphat <K₂HPO₄> oder seinem Trihydrat <K₂HPO₄•3H₂O>, Dinatriumcitrat-Dihydrat <Na₃C₆H₅O₇•2H₂O>, Trikaliumcitrat-Monohydrat <K₃C₆H₅O₇•H₂O>, Zitronensäure, Benzalkoniumchlorid, Chlorhexidindigluconat oder - dihydrochlorid, Benzethoniumchlorid, Benzoesäure, Sorbinsäure, Dehydroessigsäure, Phenoxyethanol und Benzylalkohol ausgewählt sind.

8. Pharmazeutische Zusammensetzung nach einem der vorstehenden Ansprüche, wobei die Zusammensetzung ist:
(i) Xylometazolinhydrochlorid <1>, 0,10 Gew.-%,
(ii) Natriumhyaluronat <2a>, äquivalent mit einem HA-Gehalt von 0,30 Gew.-%, und
(iii) Hilfsstoffe ausgewählt sind, um zu sein: Natriumchlorid <NaCl> 0,45 Gew.-%, Kaliumdihydrogenphosphat <KH₂PO₄> 0,825 Gew.-%, Dinatriumhydrogenphosphat-Dihydrat <Na₂HPO₄•2H₂O> 0,119 Gew.-%, und gereinigtes Wasser, bis zu 100 % der Zusammensetzung.

9. Pharmazeutische Zusammensetzung nach einem der vorstehenden Ansprüche in der abschließenden Dosierungsform eines Nasensprays zum Herstellen eines Aerosols damit, wobei die Tröpfchengrößenverteilung <DSD> sind:
A. DSD <10> = >10 µm
B. DSD <50> = 20-150 µm, und
C. DSD <90> = <250 µm.

10. Prozess zur Herstellung einer pharmazeutischen Zusammensetzung nach einem der Ansprüche 1-9, wobei der Prozess die folgenden Herstellungsschritte beinhaltet:
(i) Auflösung von Bestandteilen (i-iii) in größeren Teilen (80-90 %) gereinigten Wassers, durch Rühren bei Raumtemperatur 15-120 Minuten lang;
(ii) Zugabe des restlichen gereinigten Wassers, bis zu 100 Gew.-% der Standard-Produktionschargengröße;
(iii) Rühren, bis zur Bildung einer homogenen Lösung 1-30 Minuten lang;
(iv) Filtern der derart erhaltenen Lösung durch einen 1,2-µm-Filter zur Beseitigung von Spuren mechanischer Verunreinigungen;
(v) Sterilisieren der erhaltenen Lösung durch Filtern durch einen sterilen 0,2 µm-Filter; und
(vi) Abfüllen in sterile Behälter.

11. Pharmazeutische Zusammensetzung nach einem der Ansprüche 1-9 zur Verwendung bei der Behandlung von Rhinitis, Rhinosinusitis, Sinusitis unterschiedlicher Ätiologien, einschließlich akute virale Atemwegsinfektionen <ARVI>, Nasenverstopfung in Verbindung mit Rhinitis, Rhinosinusitis und Sinusitis und zur Wiederherstellung der mukoziliären Aktivität.

12. Pharmazeutische Zusammensetzung nach Anspruch 11, insbesondere zur Verwendung zur Behandlung von Nasenverstopfung in Verbindung mit Rhinitis, Rhinosinusitis und Sinusitis.

## Revendications

1. Composition pharmaceutique améliorée à usage nasal comprenant deux principes actifs (i) et (ii) :
(i) du chlorhydrate de xylométazoline <1>,
(ii) de l'acide hyaluronique <**2** ; HA> ou ses sels pharmaceutiquement acceptables tels que l'hyaluronate de sodium <2a ; SH> : 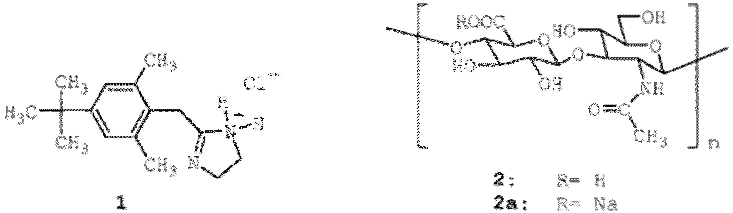 et,
(iii) un ou plusieurs excipients nécessaires à la préparation de formes posologiques finales stériles à usage nasal, sélectionnées dans le groupe consistant en : des gouttes nasales, un lavage nasal et un spray nasal destinés à un usage unique ou multiple à partir du récipient correspondant ;
dans laquelle ladite formulation présente une stabilité accrue de la xylométazoline et une activité mucociliaire accrue, une osmolalité de 0,24-0,50 Osmol/kg et la valeur du pH de ladite composition étant sélectionnée pour être de 4,0-7,5, **caractérisée en ce que** l'acide hyaluronique <2> ou son sel pharmaceutiquement acceptable 2a est utilisé sous une forme à faible poids moléculaire <LMW HA ou SH> de 150 000-250 000 Daltons.

2. Composition pharmaceutique selon la revendication 1, dans laquelle elle est **caractérisée par** une viscosité ne dépassant pas 10 mPas, mesurée par un viscosimètre rotatif à 160 tr/min à l'aide d'une broche Y dans le volume d'échantillon de 16 ml à 25 °C, pour une filtration de stérilisation efficace à travers un filtre de 0,2 µm et pour la formation de gouttelettes en cas d'utilisation comme spray nasal.

3. Composition pharmaceutique selon l'une quelconque des revendications précédentes, dans laquelle la fraction massique de chlorhydrate de xylométazoline <1> est sélectionnée pour être de 0,05-0,20 % p/p, de préférence de 0,05-0,10 % p/p.

4. Composition pharmaceutique selon l'une quelconque des revendications précédentes, dans laquelle la fraction massique d'acide hyaluronique <2> ou hyaluronate de sodium <2a> est sélectionnée pour être supérieure à 0,01 % et ne pas dépasser 1,00 % p/p, de préférence de 0,30-0,60 % p/p.

5. Composition pharmaceutique selon l'une quelconque des revendications précédentes, dans laquelle les fractions massiques desdits principes (i-iii) sont sélectionnées pour être :
(i) du chlorhydrate de xylométazoline <1>, 0,10 % p/p,
(ii) de l'hyaluronate de sodium <2a>, équivalent à une teneur en HA de 0,30-0,60 % p/p, et,
(iii) des excipients, jusqu'à 100 % de la composition.

6. Composition pharmaceutique selon l'une quelconque des revendications précédentes, dans laquelle les excipients sont sélectionnés dans le groupe comprenant de l'eau purifiée, des agents d'ajustement de la tonicité, des humectants, des agents chélateurs, des sels tampons et des conservateurs.

7. Composition pharmaceutique selon la revendication 5, dans laquelle les excipients sont sélectionnés parmi l'eau purifiée, le chlorure de sodium <NaCl>, le chlorure de potassium <KCl>, le chlorure de calcium <CaCl₂>, le chlorure de magnésium <MgCl₂>, le glycérol, le 1,2-propylène glycol, le polyéthylène glycol 200, le polyéthylène glycol 300, le polyéthylène glycol 400, l'édétate disodique dihydraté <Na₂EDTA•2H₂O>, le dihydrogénophosphate de sodium <NaH₂PO₄> ou son monohydrate <NaH₂PO₄•H₂O> ou heptahydrate <NaH₂PO₄•7H₂O>, le dihydrogénophosphate de potassium <KH₂PO₄>, l'hydrogénophosphate disodique dihydraté <Na₂HPO₄•2H₂O>, l'hydrogénophosphate dipotassique <K₂HPO₄> ou son trihydrate <K₂HPO₄•3H₂O>, le citrate disodique dihydraté <Na₃C₆H₅O₇•2H₂O>, le citrate tripotassique monohydraté <K₃C₆H₅O₇•H₂O>, l'acide citrique, le chlorure de benzalkonium, le digluconate ou dichlorhydrate de chlorhexidine, le chlorure de benzéthonium, l'acide benzoïque, l'acide sorbique, l'acide déhydroacétique, le phénoxyéthanol et l'alcool benzylique.

8. Composition pharmaceutique selon l'une quelconque des revendications précédentes, dans laquelle la composition est :
(i) du chlorhydrate de xylométazoline <1>, 0,10 % p/p,
(ii) de l'hyaluronate de sodium <2a>, équivalent à une teneur en HA de 0,30 % p/p, et
(iii) les excipients sont sélectionnés pour être : du chlorure de sodium <NaCl> 0,45 % p/p, du dihydrogénophosphate de potassium <KH₂PO₄> 0,825 % p/p, de l'hydrogénophosphate disodique dihydraté <Na₂HPO₄•2H₂O> 0,119 % p/p, et de l'eau purifiée, jusqu'à 100 % de la composition.

9. Composition pharmaceutique selon l'une quelconque des revendications précédentes, sous la forme posologique finale d'un spray nasal pour la production d'un aérosol, dans laquelle la distribution de taille de gouttelettes <DSD> est :
A. DSD <10> = >10 µm
B. DSD <50> = 20-150 µm, et
C. DSD <90> = <250 µm.

10. Procédé de préparation d'une composition pharmaceutique selon l'une quelconque des revendications 1-9, dans lequel ledit procédé inclut les étapes de fabrication suivantes :
(i) la dissolution des principes (i-iii) en grande partie (80-90 %) d'eau purifiée, en remuant à température ambiante pendant 15-120 minutes ;
(ii) l'ajout du reste de l'eau purifiée, jusqu'à 100 % p/p de la taille standard du lot de fabrication ;
(iii) l'agitation jusqu'à la formation d'une solution homogène pendant 1-30 minutes ;
(iv) la filtration de la solution ainsi obtenue à travers un filtre de 1,2 µm pour éliminer les traces d'impuretés mécaniques ;
(v) la stérilisation de la solution obtenue par filtration à travers un filtre stérile de 0,2 µm ; et
(vi) le remplissage dans des récipients stériles.

11. Composition pharmaceutique selon l'une quelconque des revendications 1-9, destinée à être utilisée dans le traitement de la rhinite, de la rhinosinusite, de la sinusite de différentes étiologies incluant l'infection virale respiratoire aiguë <ARVI>, de la congestion nasale liée à la rhinite, à la rhinosinusite et à la sinusite, et pour rétablir l'activité mucociliaire.

12. Composition pharmaceutique selon la revendication 11, destinée à être utilisée en particulier pour le traitement de la congestion nasale liée à la rhinite, à la rhinosinusite et à la sinusite.
